# EUROPÄISCHE PATENTSCHRIFT

(11) **EP 0 456 062 B1**
(45) Veröffentlichungstag und Bekanntmachung des Hinweises auf die Patenterteilung: **05.03.1997**
(21) Anmeldenummer: 91106869.0
(22) Anmeldetag: 27.04.1991
(51) Int. Cl.: C07D 251/34, C08G 18/79, C08G 18/32

(54) **Verfahren zur Herstellung von Isocyanuratpolyisocyanaten, die nach diesem Verfahren erhaltenen Verbindungen und ihre Verwendung**
Process for preparation of isocyanuratepolyisocyanates, the compounds prepared according to this process, and their use
Procédé pour la préparation des isocyanuratespolyisocyanates, les composés préparés selon ce procédé et leur utilisation

(30) Priorität: 11.05.1990 DE 4015155
(43) Veröffentlichungstag der Anmeldung: 13.11.1991
(73) Patentinhaber: BAYER AG, 51368 Leverkusen (DE)
(72) Erfinder: Pedain, Josef, Dr., W-5000 Köln 80 (DE)

(56) Entgegenhaltungen:
- EP-A- 0 155 559
- EP-A- 0 336 205
- PATENT ABSTRACTS OF JAPAN vol. 10, no. 350 (C-387)(2406) 26. November 1986 & JP-A-61151179 (MITSUI TOATSU CHEM INC.) 9. Juli 1986
- PATENT ABSTRACTS OF JAPAN vol. 9, no. 222 (C-302)(1945) 9. September 1985 & JP-A-60084274 (TOA GOSEI KAGAKU KOGYO K.K) 13. Mai 1985

## Beschreibung

Die vorliegende Erfindung betrifft ein neues Verfahren zur Herstellung von Urethan- und Estergruppen enthaltenden Isocyanuratpolyisocyanaten (Isocyanuratgruppen aufweisende Polyisocyanaten), die nach diesem Verfahren erhaltenen Verbindungen und ihre Verwendung als Polyisocyanatkomponente bei der Herstellung von Polyurethankunststoffen und insbesondere in Zweikomponenten-Polyurethanlacken.

Aliphatische Isocyanuratpolyisocyanate, insbesondere solche auf der Basis 1,6-Diisocyanatohexan (nachstehend "HDI" genannt) haben großtechnische Bedeutung erlangt. Sie werden vor allem als Polyisocyanatkomponente in Zweikomponenten-Polyurethanlacken aber auch zur Herstellung von unter dem Einfluß von Feuchtigkeit härtenden Einkomponenten-Polyurethan-Bindemitteln oder, in mit Blockierungsmitteln für Isocyanatgruppen blockierter Form, in Hitze-vernetzbaren Polyurethanlacken eingesetzt. Diese Lacke dienen überwiegend zur Lackierung von wenig flexiblen Substraten wie Metall und Holz und zeichnen sich durch hohe Lichtechtheit und Witterungsbeständigkeit, hohe Härte und sehr gute Haftung aus. Speziell Isocyanuratgruppen enthaltende, auf HDI basierende Polyisocyanate zeichnen sich gegenüber entsprechenden Biurettypen, die ebenfalls großtechnisch genutzt werden, durch eine stark verbesserte Vergilbungs- und Chemikalienbeständigkeit z.B. Teerfleckenbeständigkeit aus.

Die chemischen Grundlagen der verschiedenen Polyurethanlacke werden u.a. in "Lackkunstharze" von Hans Wagner und Hans Friedrich Sarx, Carl Hanser Verlag, München 1971, Seiten 153 bis 173 und im "Lehrbuch der Lacke und Beschichtungen" Band 1, Teil 2 von Hans Knittel, Verlag W.A. Colomb, Berlin-Oberschwandorf 1973, Seiten 512 bis 612 beschrieben.

Die Herstellung von Isocyanuratpolyisocyanaten ist beispielsweise in GB-PS 920 080, DE-AS 1 667 309, DE-OS 3 100 262, DE-OS 3 219 608, DE-OS 3 240 613, DE-OS 3 811 350, EP-A-10 589, EP-A-57 653, EP-A-89 297, EP-A-187 105 oder in der Japanischen Patentanmeldung Sho 59-271 970 vom 25.12.1984, veröffentlicht als Sho 61-151 179 am 9.7.1986 beschrieben, wobei in einigen dieser Vorveröffentlichungen auch die Mitverwendung von unterschüssigen Mengen an Hydroxylgruppen aufweisenden Verbindungen erwähnt wird. So beschreibt beispielsweise die DE-AS 1 667 309 die Herstellung von Isocyanuratpolyisocyanaten unter Verwendung von Hydroxylgruppen aufweisenden Verbindungen als Co-Katalysatoren. Die DE-OS 3 219 608 beschreibt die Mitverwendung von mehrwertigen Alkoholen eines unter 3000 liegenden Molekulargewichts in einer Menge von bis zu 15 Mol-%, bezogen auf eingesetztes HDI, bei der Herstellung von Isocyanuratpolyisocyanaten auf Basis dieses Ausgangsdiisocyanats. Zu den gemäß dieser Vorveröffentlichung geeigneten, mehrwertigen Alkoholen gehören auch nicht näher spezifizierte Polyesterpolyole. Beim Verfahren der EP-A-155 559 werden niedermolekulare Diole mit seitenständigen Alkylresten als Modifizierungsmittel mitverwendet. In der genannten Japanischen Patentveröffentlichung werden einwertige aliphatische Alkohole mit 6 bis 9 Kohlenstoffatomen als Cokatalysatoren mitverwndet.

Die Modifizierung des Ausgangsdiisocyanats mit bestimmten Estergruppen aufweisenden Diolen gemäß DE-OS 3 811 350 dient insbesondere der Erhöhung der Elastizität der aus den modizierten Polyisocyanaten hergestellten Lacküberzüge, wobei auch eine gewisse Verbesserung der Verträglichkeit der modifizierten Polyisocyanate erzielt wird.

Allen bekannten Verfahren zur Herstellung von Isocyanuratpolyisocyanaten auf Basis von aliphatischen Diisocyanaten, insbesondere auf Basis von HDI ist indessen gemeinsam, daß die jeweiligen Verfahrensprodukte den Anforderungen der Praxis bezüglich niedriger Viskosität einerseits und Verträglichkeit mit Hydroxylgruppen aufweisenden Lackharzen und physiologisch weitgehend unbedenklichen Lösungsmitteln andererseits noch nicht voll entsprechen. Die Herstellung von besonders niedrigviskosen, Isocyanuratgruppen aufweisenden Polyisocyanaten auf Basis von HDI war im übrigen bislang nur bei einem besonders geringen Umsatz von etwa 10 bis 20 % des eingesetzten HDI möglich. Derartige Verfahren, bei welchen die Trimerisierungsreaktion bereits bei einem sehr niedrigen Trimerisierungsgrad abgebrochen werden und das überschüssige HDI destillativ entfernt werden muß, sind naturgemäß aufwendig und teuer.

Es war daher die der Erfindung zugrunde liegende Aufgabe, neue Isocyanuratpolyisocyanate auf Basis von aliphatischen Diisocyanaten zur Verfügung zu stellen, die sich gleichzeitig durch die Vorteile einer niedrigen Viskosität und einer verbesserten Verträglichkeit mit den Üblichen, Hydroxylgruppen aufweisenden Lackharzen bzw. den üblichen, physiologisch weitgehend unbedenklichen Lösungsmitteln auszeichnen, und die wirtschaftlich vorteilhaft bei einem hohen Umsatz des eingesetzten Diisocyanats herstellbar sind.

Diese Aufgabe konnte durch die Bereitstellung des nachstehend näher beschriebenen erfndungsgemäßen Verfahrens gelöst werden.

Gegenstand der Erfindung ist ein Verfahren zur Herstellung von Isocyanuratpolyisocyanaten durch Trimerisierung eines Teils der Isocyanatgruppen von (cyclo)aliphatischen Diisocyanaten in Gegenwart von die Trimerisierung von Isocyanatgruppen beschleunigenden Katalysatoren, Abbruch der Trimerisierungsreaktion beim jeweils gewünschten Trimerisierungsgrad und Entfernung von nicht umgesetztem Ausgangsdiisocyanat und von gegebenenfalls weiteren flüchtigen Bestandteilen, dadurch gekennzeichnet, daß man dem Reaktionsgemisch zu einem beliebigen Zeitpunkt vor Entfernung des überschüssigen Ausgangsdiisocyanats mindestens einen einwertigen, Estergruppen aufweisenden Alkohol des Molekulargewichts 146 bis 412 der allgemeinen Formel 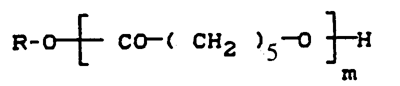 in welcher
- R: für einen, gegebenenfalls Ethersauerstoffatome aufweisenden, gegebenenfalls olefinisch ungesättigten (cyclo)aliphatischen Kohlenwasserstoffrest mit 1 - 18 Kohlenstoffatomen steht und
- m: für eine ganze oder im statistischen Mitteln gebrochene Zahl von 1 bis 2 steht und
in einer Menge von 1 bis 30 Gew.-%, bezogen auf das Gewicht des als Ausgangsmaterial eingesetzten Diisocyanats hinzufügt und mit einem Teil der vorliegenden Isocyanatgruppen unter Urethanbildung abreagieren läßt, wobei die Art und Mengenverhältnis der Reaktionspartner im übrigen so gewählt werden, daß in dem Reaktionsgemisch ohne Einbeziehung von gegebenenfalls mitverwendetem inerten Lösemittel nach beendeter Umsetzung noch mindestens 10 Gew.-% an freiem Ausgangsdiisocyanat vorliegen.

Gegenstand der Erfindung sind auch die nach diesem Verfahren erhaltenen Isocyanuratpolyisocyanate.

Gegenstand der Erfindung ist schließlich auch die Verwendung der nach diesem Verfahren erhaltenen Isocyanuratpolyisocyanate, gegebenenfalls in mit Blockierungsmitteln für Isocyanatgruppen blockierter Form, zur Herstellung von Polyurethankunststoffen, insbesondere in Zweikomponenten-Polyurethanlacken.

Ausgangsmaterialien für das erfindungsgemäße Verfahren sind (i) (cyclo)aliphatische Diisocyanate und (ii) ausgewählte, einwertige Polyesteralkohole.

Bei den als Ausgangsdiisocyanat (i) eingesetzten (cyclo)aliphatischen Diisocyanaten handelt es sich um organische Diisocyanate mit aliphatisch und/oder cycloaliphatisch gebundenen Isocyanatgruppen. Typische Beispiele sind 1,6-Diisocyanatohexan (HDI), 1-Isocyanato-3,3,5-trimethyl-5-isocyanatomethyl-cyclohexan (IPDI) oder 4,4'-Diisocyanato-dicyclohexylmethan. Andere (cyclo)aliphatische Diisocyanate als HDI werden allerdings allenfalls als Abmischkomponente mit HDI verwendet. Dies bedeutet, daß es sich bei den erfindungsgemäß zum Einsatz gelangenden Ausgangsdiisocyanaten entweder um HDI oder um Gemische von HDI mit anderen (cyclo)aliphatischen Diisocyanaten der beispielhaft genannten Art handelt, mit der Maßgabe, daß in den Gemischen mindestens 30 Mol-%, vorzugsweise mindestens 70 Mol-% HDI vorliegen. Ganz besonders bevorzugt wird HDI als alleiniges Ausgangsdiisocyanat verwendet.

Die erfindungsgemäß als Ausgangsmaterialien einzusetzenden Diisocyanate können in technischer Reinheit verwendet werden. Ganz besonders bevorzugt wird jedoch ein von Kohlendioxid weitgehend befreites HDI als alleiniges Ausgangsdiisocyanat verwendet, da mit dessen Verwendung eine besonders schonend ablaufende Trimerisierungsreaktion unter Verwendung von minimalen Mengen an Katalysatoren möglich ist.

Das besonders bevorzugt als Ausgangsdiisocyanat zum Einsatz gelangende HDI weist einen Gehalt an Kohlendioxid von weniger als 20 ppm (Gewicht), vorzugsweise von weniger als 10 ppm (Gewicht) und besonders bevorzugt von weniger als 5 ppm (Gewicht) auf.

Technisches, durch Destillation gereinigtes HDI, wie es bislang auch zur Herstellung von Isocyanuratgruppen aufweisenden Polyisocyanaten eingesetzt wurde, weist beträchtliche Mengen (ca. 20 ppm bis 100 ppm (Gewicht)) an Kohlendioxid auf.

Kohlendioxid kann bei der Herstellung in das HDI gelangen, beispielsweise bei der Phosgenierung von Kohlensäuresalzen des Hexamethylendiamins. Es kann beim Lagern aus der Luft aufgenommen werden, und es kann durch chemische Reaktion der NCO-Gruppen, z.B. durch Carbodiimidbildung oder mit einer Spur Feuchtigkeit, entstehen. Frisch durch Vakuumdestillation gereinigtes HDI enthält nach 24 Stunden im verschlossenen Gefäß beispielsweise 40 ppm Kohlendioxid. Über einen Zeitraum von ca. 6 Monaten gelagertes HDI kann, falls das Gebinde während der Lagerzeit geöffnet wurde, bis zu 0,6 Gew.-% Kohlendioxid enthalten.

Die Entfernung von Kohlendioxid aus HDI kann durch Ausblasen mit Reinstickstoff oder Edelgas, zum Beispiel mit Argon, erfolgen, z.B. bei 0 - 70°C. Auch eine höhere Temperatur kann angewendet werden, bietet aber keine entscheidenden Vorteile.

Es ist natürlich ebenfalls möglich und stellt ebenfalls eine bevorzugte Variante dar, zunächst HDI mit einem höher als 20 ppm liegenden Gehalt an Kohlendioxid mit unterschüssigem Esteralkohol erfindungsgemäß zu modifizieren, dann das gelöste Kohlendioxid weitgehend zu entfernen, und schließlich die Trimerisierungsreaktion durchzuführen.

Bei den einwertigen Esteralkoholen (ii) handelt es sich um Verbindungen der Formel 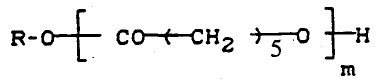 für welche
- R und m: die bereits oben genante Bedeutung haben.

Vorzugsweise werden solche Esteralkohole der allgemeinen Formel eingesetzt, für welche
- R: für einen gesättigten aliphatischen Kohlenwasserstoffrest mit 1 bis 12 Kohlenstoffatomen steht und
- m: für eine ganze oder gebrochene Zahl von 1 bis 2 steht.

Das Molekulargewicht dieser einwertigen Esteralkohole liegt bei ca. 146 - 412, vorzugsweise 160 - 300.

Die Herstellung der einwertigen Esteralkohole erfolgt durch Umsetzung der entsprechenden Estergruppen-freien Alkohole R-OH mit Caprolacton. Diese an sich bekannte Umsetzung kann beispielsweise in Gegenwart von Bortrifluorid-Etherat oder organischen Zinnverbindungen wie z.B. Zinndioctoat als Katalysatoren bei 120 bis 200° C durchgeführt werden.

Bei den einwertigen Esteralkoholen (ii) handelt es sich im allgemeinen um Gemische, da die Anlagerung des Lactons an die Alkohole nicht zu einheitlichen Produkten führt sondern, den gemachten Angaben bezüglich m entsprechend, zu Gemischen in denen Einzelverbindungen mit unterschiedlichen Werten für m vorliegen. Außerdem können schon deshalb Gemische vorliegen, weil bei der Herstellung der Esteralkohole selbstverständlich auch Gemische unterschiedlicher Estergruppen-freier Alkohole der beispielhaft genannten Art und/oder Gemische unterschiedlicher Lactone der beispielhaft genannten Art zum Einsatz gelangen können. Da es sich bei m um einen statistischen Mittelwert handelt, können in den Gemischen, selbst wenn m im statistischen Mittel für 1 oder eine Zahl größer als 1 steht noch gewisse Anteile an Estergrupppen-freien Alkoholen R-OH vorliegen.

Das erfindungsgemäße Verfahren kann im Prinzip in völliger Analogie zu den bekannten Verfahren zur Herstellung von Isocyanuratpolyisocyanaten durchgeführt werden. Dies bedeutet insbesondere, daß die bekannten Trimerisierungskatalysatoren des Standes der Technik, wie sie beispielsweise in den obengenannten Literaturstellen empfohlen werden, verwendet werden können.

Vorzugsweise werden beim erfindungsgemäßen Verfahren quaternäre Ammoniumhydroxide als Katalysator eingesetzt. Grundsätzlich geeignet sind alle beliebigen quaternären Ammoniumhydroxide, wie sie bereits früher als Trimerisierungskatalysatoren für Isocyanatgruppen empfohlen worden sind. Geeignet sind beispielsweise die quaternären Ammoniumhydroxide gemäß US-PS 3 487 080, Kolonne 2, Zeilen 10 bis 38 oder gemäß EP-A-10 589, Seite 6, Zeile 5 bis Seite 8, Zeile 10. Gut geeignet sind auch Verbindungen der Formel 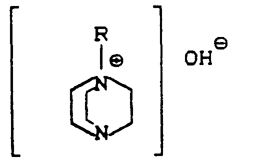 in welcher
- R: für einen Alkylrest mit 1 bis 20, vorzugsweise 1 bis 4 Kohlenstoffatomen, einen araliphatischen Kohlenwasserstoffrest mit 7 bis 10, vorzugsweise 7 Kohlenstoffatomen oder einen gesättigten cycloaliphatischen Kohlenwasserstoffrest mit 4 bis 10, vorzugsweise 5 bis 6 Kohlenstoffatomen, steht.

Zu den bevorzugten Katalysatoren gehören Verbindungen der allgemeinen Formel 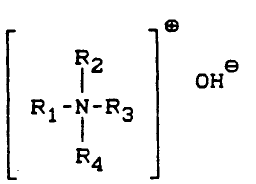 für welche
- R₁, R₂ und R₃: für gleiche oder verschiedene Alkylreste mit 1 bis 18, insbesondere 1 bis 4 Kohlenstoffatomen, besonders bevorzugt für Methylgruppen stehen und
- R₄: für einen Benzyl-, 2-Hydroxyethyl-, 2-Hydroxypropyl- oder einen 2-Hydroxybutyl-Rest steht.

Besonders bevorzugte Katalysatoren sind N,N,N-Trimethyl-N-benzylammoniumhydroxid und N,N,N-Trimethyl-N-(2-hydroxypropyl)-ammoniumhydroxid.

Die jeweils optimale Menge an Katalysator hängt von der Natur des Katalysators ab und kann mit Hilfe eines orientierenden Vorversuchs ermittelt werden. Die Menge des eingesetzten Katalysators liegt beim erfindungsgemäßen Verfahren, bezogen auf eingesetztes Ausgangsdiisocyanat im allgemeinen unterhalb 1 Gew.-%. Bei Verwendung von HDI als Ausgangsdiisocyanat, welches entsprechend den oben gemachten Ausführungen weitgehend von Kohlendioxid befreit worden ist, und unter Verwendung der bevorzugten Ammoniumhydroxid-Katalysatoren liegt die Katalysatormenge bei weniger als 0,03 Gew.-%, vorzugsweise bei weniger als 0,01 Gew.-% und besonders bevorzugt bei 0,0005 bis 0,005 Gew.-%, bezogen auf eingesetztes HDI.

Die Katalysatoren können lösungsmittelfrei benutzt werden, sie werden aber bevorzugt in verdünnter Lösung eingesetzt. Geeignete Lösungsmittel sind in den zitierten Veröffentlichungen beschrieben.

Trimerisierungs- und Urethanisierungsreaktion werden vorzugsweise lösemittelfrei durchgeführt, was die Mitverwendung von üblichen Lacklösungsmitteln, beispielsweise von Estern wie Butylacetat oder Ethoxyethylacetat, Ketonen wie Methyl-isobutyl-keton oder Methylethylketon oder Kohlenwasserstoffen wie Xylol bzw. von Gemischen derartiger Lösungsmitteln jedoch nicht ausschließt. Da im Anschluß jedoch nicht umgesetztes Ausgangsdiisocyanat entfernt wird, würde die Mitverwendung derartiger Lösungsmittel lediglich auf einen überflüssigen, zusätzlichen Aufwand hinauslaufen.

Zur Beendigung der Trimerisierungsreaktion wird im allgemeinen der Katalysator thermisch desaktiviert und/oder dem Reaktionsgemisch zur Desaktivierung des Katalysators ein geeignetes Katalysatorgift zugefügt. Geeignete Katalysatorengifte sind insbesondere im Falle der Verwendung der bevorzugten Ammoniumhydroxid-Katalysatoren anorganische Säuren wie Salzsäure, phosphorige Säure oder Phosphorsäure, Sulfonsäuren oder ihre Derivate wie Methansulfonsäure, p-Toluolsulfonsäure, p-Toluolsulfonsäuremethyl- oder - ethylester oder perfluorierte Sulfonsäuren wie beispielsweise Nonafluorbutansulfonsäure. Besonders gut geeignete Desaktivatoren, d.h. Katalysatorengifte sind saure Ester der phosphorigen Säure oder der Phosphorsäure wie beispielsweise Dibutylphosphit, Dibutylphosphat oder Di-(2-ethylhexyl)-phosphat, die vorzugsweise in Form einer verdünnten Lösung in HDI eingesetzt werden. Die Desaktivatoren werden dem Reaktionsgemisch im allgemeinen in einer dem Katalysator zumindest äquivalenten Menge zugefügt. Da sich die Katalysatoren jedoch während der Trimerisierungsreaktion teilweise zersetzen können, ist oftmals die Zugabe einer unteräquivalenten Menge des Desaktivators ausreichend. Bei Verwendung von thermisch labilen Katalysatoren, beispielsweise von quaternären Ammoniumhydroxiden mit Hydroxyalkyl-Substituenten am Stickstoff, kann oftmals auch auf die Zugabe eines Katalysatorengifts völlig verzichtet werden, da bei Verwendung derartiger Katalysatoren oftmals ein Abbruch der Reaktion durch kurzzeitiges Erhitzen des Reaktionsgemischs auf oberhalb 100°C liegende Temperaturen ausreicht (thermische Zersetzung, d.h. Desaktivierung des Katalysators).

Andererseits ist zwecks Gewährleistung einer sicheren Abstoppung der Reaktion oftmals auch die Verwendung einer größeren als der äquivalenten Menge, beispielsweise einer doppelt äquivalenten Menge des Desaktivators zweckmäßig. Vorzugsweise wird somit unter Verwendung von Desaktivatoren (Katalysatorengiften) in bis zu 2-fach äquivalenten Mengen, bezogen auf die Menge des eingesetzten Katalysators, gearbeitet.

Der erfindungswesentliche Punkt besteht nun darin, neben der Trimerisierung eines Teils der Isocyanatgruppen des Ausgangsdiisocyanats einen weiteren Teil dieser Isocyanatgruppen durch Urethanisierung mit den beispielhaft genannten Esteralkoholen zu modifizieren. Hierbei ist es unerheblich, in welcher Reihenfolge Urethanisierung und Trimerisierung erfolgen mit der Einschränkung, daß beide Verfahrensschritte vor Entfernung des überschüssigen Ausgangsdiisocyanats erfolgen müssen. Dies bedeutet, daß die Urethanisierungsreaktion mit dem Esteralkohol unter Verbrauch eines Teils der Isocyanatgruppen vor Zugabe des Trimerisierungskatalysatars erfolgen kann. Die Urethanisierung kann auch mit nur einem Teil des überschüssigen Diisocyanats erfolgen und weiteres Diisocyanat kann vor der nachfolgenden Trimerisierungsreaktion noch zugesetzt werden. Urethanisierung und Trimerisierung können gleichzeitig durchgeführt werden, indem Esteralkohol und Trimerisierungskatalysator gleichzeitig, beispielsweise im Gemisch zugesetzt werden. Die Urethanisierung kann beginnen, bevor die Trimerisierung ganz abgeschlossen ist, sie kann auch nach abgeschlossener Trimerisierung eingeleitet werden.

Der Esteralkohol kann auch in Teilmengen zu einem beliebigen Zeitpunkt des Verfahrens zugesetzt werden. Trimerisierung und Urethanisierung sollten abgeschlossen sein, bevor man beginnt, überschüssiges Ausgangsdiisocyanat zu entfernen.

Bezüglich der Mengenverhältnisse der einzelnen Reaktionspartner ist zu sagen, daß die Esteralkohole im allgemeinen in einer Menge von 1-30 Gew.-%, vorzugsweise von 5-20 Gew.-%, bezogen auf das Gewicht des als Ausgangsmaterial eingesetzten Diisocyanats zur Anwendung gelangen, wobei jedoch vorzugsweise dafür Sorge zu tragen ist, daß das Ausgangsdiisocyanat stets in einem solchen Überschuß eingesetzt wird, daß nach erfolgter Umsetzung in dem Reaktionsgemisch noch mindestens 10 Gew.-%, vorzugsweise 35 bis 70 Gew.-%, bezogen auf Gesamtgemisch ohne Einbeziehung von gegebenenfalls mitverwendeten inerten Lösungsmittel, an freiem Ausgangsdiisocyanat vorliegen, wobei das Molverhältnis von Isocyanuratgruppen zu Urethangruppen in den von überschüssigem Ausgangsdiisocyanat befreiten Verfahrensprodukten vorzugsweise bei 40:1 bis 5:1 liegen sollte.

Das erfindungsgemäße Verfahren wird im allgemeinen innerhalb des Temperaturbereichs von 0 bis 150°C durchgeführt, wobei die gegebenenfalls separat am Anfang oder am Ende durchgeführte Urethanisierung vorzugsweise bei 20 bis 150°C, insbesondere 80 bis 130°C und die gegebenenfalls separat vor oder nach der Urethanisierung erfolgende Trimerisierung vorzugsweise innerhalb des Temperaturbereichs von 0 bis 100°C und insbesondere von 20 bis 80°C erfolgt. Falls beide Reaktionsschritte gleichzeitig durchgeführt werden, liegt die Reaktionstemperatur im allgemeinen bei 0 bis 100°C, vorzugsweise 40 bis 80°C. Der Abbruch der Trimerisierungsreaktion erfolgt, wie dargelegt, thermisch und/oder durch Zugabe eines Katalysatorengifts, vorzugsweise nach Erreichen eines Trimerisierungsgrads von 10 bis 40 %, insbesondere 20 bis 30 %, wobei unter "Trimerisierungsgrad" der Prozentsatz der Isocyanatgruppen des Ausgangsdiisocyanats zu verstehen ist, die unter Trimerisierung abreagieren, wobei bei dieser Berechnung die Urethanbildungsreaktion unberücksichtigt bleibt. Wesentlich ist jedoch, wie oben bereits ausgeführt, daß nach beendeter Urethanisierung und Trimerisierung noch mindestens 10 Gew.-% unumgesetztes Ausgangsdiisocyanat im Reaktionsgemisch vorliegen.

Nach beendeter Urethanisierung und Trimerisierung wird das überschüssige Ausgangsdiisocyanat gegebenenfalls zusammen mit anderen, im Reaktionsgemisch vorliegenden flüchtigen Bestandteilen wie gegebenenfalls mitverwendete Lösungsmittel durch eine geeignete Maßnahme bis auf einen Restgehalt an Ausgangsdiisocyanat von maximal 0,5 Gew.-% abgetrennt. Man kann dies durch eine Dünnschichtdestillation oder Extraktion, beispielsweise mittels n-Hexan als Extraktionsmittel, erreichen.

Die erfindungsgemäßen Urethan- und Isocyanuratgruppen enthaltenden Verfahrensprodukte sind flüssige, praktisch farblose Polyisocyanate. Die erfindungsgemäßen Verfahrensprodukte auf Basis von HDI weisen im allgemeinen einen NCO-Gehalt von 10 bis 20 Gew.-% auf. Ihre HAZEN-Farbzahl (DIN 53 409) liegt unter 100, im allgemeinen unter 50. Ihre Viskosität bei 23°C liegt im allgemeinen unter 5000, vorzugsweise unter 1500 mPa.s. Unter Zugrundelegung eines vergleichbaren Umsatzes des eingesetzten Ausgangsisocyanats liegt die Viskosität der erfindungsgemäßen Verfahrensprodukte wesentlich unter der Viskosität von entsprechenden, ohne Mitverwendung der erfindungswesentlichen Esteralkohole hergestellten, Isocyanuratgruppen aufweisenden Polyisocyanaten.

Die erfindungsgemäßen Verfahrensprodukte sind in üblichen Lösungsmitteln wie Estern, Ketonen und Kohlenwasserstoffen löslich, lassen sich damit ohne Eintrübung verdünnen und zeichnen sich durch gute Lagerstabilität aus. Sie sind weitgehend frei von Nebenprodukten. Sie eignen sich in hervorragender Weise als Härter für Zweikomponenten-Polyurethanlacke, in denen als Polyhydroxylverbindungen die üblichen Polyetherpolyole, Polyesterpolyole und/oder Polyacrylatpolyole als Reaktionspartner für die Polyisocyanate vorliegen. Besonders bevorzugte Reaktionspartner für die erfindungsgemäßen Verfahrensprodukte sind Hydroxylgruppen aufweisende Polyacrylate, d.h. Polymerisate bzw. Copolymerisate von (Meth)acylsäure-alkylestern, gegebenenfalls mit Styrol oder anderen coolymerisierbaren olefinisch ungesättigten Monomeren.

Den Zweikomponentenpolyurethanlacken, die als Bindemittel Kombinationen derartiger Polyhydroxylverbindungen mit den erfindungsgemäßen Verfahrensprodukten als Härter enthalten, können selbstverständlich die aus der Lacktechnologie üblichen Hilfs- und Zusatzmittel wie Pigmente, Verlaufsmittel, Katalysatoren, Lösungsmittel u.dgl. einverleibt werden. Die Zweikomponenten-Polyurethanlacke, die die erfindungsgemäßen Verfahrensprodukte als Härter enthalten, härten bei Raumtemperatur oder wenig erhöhter Temperatur zu chemikalienbeständigen Lackfilmen aus.

Selbstverständlich ist es auch möglich, die erfindungsgemäßen Verfahrensprodukte in mit Blockierungsmitteln blockierter Form als Härter in Hitze-vernetzbaren Einkomponentenlacken zu verwenden. Als Blockierungsmittel eignen sich die hierfür üblichen Verbindungen wie beispielsweise Phenol, Kresole, Trimethylphenole, tert.-Butylphenole, tertiäre Alkohole wie tert.-Butanol, tert.-Amylalkohol, Dimethylphenylcarbinol; leicht Enole bildende Verbindungen wie Acetessigsäureethylester, Acetylaceton, Malonsäurediethylester; sekundäre aliphatische und aromatische Amine wie Dibutylamin, N-Methylanilin, die N-Methyltoluidine, N-Phenyltoluidin, N-Phenylxylidin; Imide wie Succinimid; Lactame wie ε-Caprolactam, δ-Valerolactam; Oxime wie Butanonoxim, Cyclohexanonoxim; Mercaptane wie Methylmercaptan, Ethylmercaptan, Butylmercaptan, 2-Mercaptobenzthiazol, α-Naphthylmercaptan, Dodecylmercaptan oder Triazole wie 1H-1,2,4-Triazol.

Die erfindungsgemäßen Verfahrensprodukte können auch mit Polyaminen kombiniert werden, deren Aminogruppen blockiert sind wie z.B. mit Polyketiminen, Polyaldiminen oder Oxazolanen. Unter dem Einfluß von Feuchtigkeit entstehen freie Aminogruppen und (im Falle der Oxazolane) freie OH-Gruppen, die unter Vernetzung mit den NCO-Gruppen abreagieren.

In allen genannten Lackkombinationen liegen die Polyisocyanatkomponente und der Reaktionspartner in solchen Mengen vor, daß auf jede (gegebenenfalls blockierte) NCO-Gruppe 0,8 bis 3, vorzugsweise 0,9 bis 1,8 (gegebenenfalls blockierte) gegenüber Isocyanaten reaktionsfähige Gruppen entfallen.

Die die erfindungsgemäßen Verfahrensprodukte, gegebenenfalls in blockierter Form, als Härter enthaltenden Beschichtungsmittel eignen sich zur Beschichtung beliebiger Substrate. Sie zeichnen sich gegenüber analogen Beschichtungsmitteln, die als Härter die üblichen Polyisocyanate enthalten, durch eine erhöhte Flexibilität der Beschichtungen aus. Das besonders bevorzugte Anwendungsgebiet für die erfindungsgemäßen Verfahrensprodukte besteht in ihrer Verwendung als Härter für Zweikomponenten-Kunststofflacke auf der Basis der oben beispielhaft genannten Polyhydroxylverbindungen, insbesondere bei der Lackierung von flexiblen Kunststoffteilen.

Die die erfindungsgemäßen Polyisocyanate enthaltenden Lacke ergeben Filme, die aber auch überraschend gut auf metallischem Untergrund haften, besonders lichtecht, wärmeformstabil und sehr abriebfest sind. Darüber hinaus zeichnen sie sich durch große Härte, Elastizität, sehr gute Chemikalienbeständigkeit, hohen Glanz, ausgezeichnete Wetterbeständigkeit und gute Pigmentierbarkeit aus.

In den nachfolgenden Beispielen beziehen sich alle Prozentangaben auf Gewichtsprozente.

### Beispiel 1

### Herstellung des Ausgangsmaterials (Estergruppen enthaltender Alkohol)

130 g (1 Mol) 2-Ethylhexanol werden mit 114 g (1 Mol) Caprolacton versetzt und vermischt und dann nach Zugabe von 0,024 g Zinn-dioctoat 5 h bei 160°C unter Rühren zur Reaktion gebracht. Man erhält ein flüssiges, farbloses Produkt mit einer Hydroxylzahl von 230. Im Gaschromatogramm erkennt man, daß etwa 10 Gew.-% des Ausgangsalkohols nicht umgesetzt sind. Freies Caprolacton ist nicht mehr zugegen. Neben der Hauptsubstanz, dem Estergruppen-haltigen Alkohol aus 1 Mol 2-Ethylhexanol und 1 Mol Caprolacton sind auch höhermolekulare Anteile zugegen.

### Beispiel 2

Das Beispiel beschreibt die erfindungsgemäße Herstellung eines niedrigviskosen Isocyanuratgruppen enthaltenden Polyisocyanats bei einem mittleren Umsatz.

1680 g HDI werden mit 24,4 g Esteralkohol, wie er nach Beispiel 1 erhalten wurde, 3 h bei 100°C erhitzt. Während der Urethanisierungsreaktion wird ein Strom von Reinstickstoff durch die Reaktionsmasse geleitet und dadurch gelöstes Kohlendioxid entfernt.

Man läßt danach abkühlen auf 45°C und gibt dann als Katalysator tropfenweise 20 g einer 0,5 %igen Lösung von Benzyltrimethylammoniumhydroxid in 2-Ethylhexanol zu. Die exotherme Trimerisierungsreaktion beginnt sofort. Man läßt die Temperatur bei etwa 55 bis 60°C und leitet weiter einen schwachen Stickstoffstrom durch die Reaktionsmasse. Nach etwa 7 h ist ein NCO-Gehalt von 40,7 % erreicht. Man unterbricht die Reaktion durch Zugabe von 0,3 g einer 25 %igen Lösung von Dibutylphosphat in HDI. Man kühlt ab auf 25°C und entfernt überschüssiges HDI durch Destillation in einem Kurzwegverdampfer bei 130°C. Man erhält 570 g eines flüssigen, klaren Polyisocyanats mit folgenden Daten:
Viskosität: 1140 mPa.s/23°C
NCO-Gehalt: 21,5 %
Hazen-Farbzahl: 20
freies HDI: 0,01

Führt man den Versuch durch, verzichtet dabei auf die Zugabe des Estergruppen-haltigen Monoalkohols und unterbricht die Trimerisierung bei ca. 41 % NCO, so erhält man ein Produkt mit einer Viskosität von 3500 mPa.s/23°C.

### Beispiel 3

Dieses Beispiel beschreibt die Herstellung eines besonders niedrigviskosen Isocyanurat-Polyisocyanats.

Man verfährt wie in Beispiel 2 und setzt zunächst 1680 g HDI mit 61 g Monoalkohol aus Beispiel 1 um. Man polymerisiert mit 15 g der Katalysatorlösung und unterbricht durch Zugabe von Dibutylphosphat-Lösung bei einem NCO-Gehalt von ca. 41 %.

Man erhält nach Entfernung von überschüssigem HDI 554 g eines Polyisocyanats mit folgenden Daten.
NCO-Gehalt: 20,0 %
Viskosität: 810 mPa.s/23°C
freies HDI: 0,01 %
Hazen-Farbzahl: 30

### Beispiel 4

Dieses Beispiel beschreibt die Herstellung eines erfindungsgemäßen Polyisocyanats mit sehr hoher Ausbeute und noch verhältnismäßig niedriger Viskosität.

Man verfährt wie in Beispiel 2 und benutzt die gleichen Mengen an Ausgangsmaterialien wie in Beispiel 3. Man unterbricht die Reaktion aber erst bei einem NCO-Gehalt im Rohprodukt von 31,7 %. Nach Entfernung von freiem HDI erhält man 1030 g eines Produkts mit folgenden Daten:
Viskosität: 4850 mPa.s/23°C
NCO-Gehalt: 18,8 %
freies HDI: 0,01 %
Hazen-Farbzahl: 30

### Beispiel 5

Dieses Beispiel beschreibt ein Produkt, das mit hoher Ausbeute und niedriger Viskosität erhalten wird. Man verfährt wie in Beispiel 2.

Ansatzmengen:
- 1680 g: HDI
- 122 g: Monoalkohol aus Beispiel 1
- 25 g: der 0,5 %igen Katalysatorlösung
- 0,5 g: 25 %ige Lösung von Dibutylphosphat in HDI.

Nach Entfernung von überschüssigem HDI erhält man 1073 g eines klaren, flüssigen Polyisocyanates mit folgenden Daten:
Viskosität: 3300 mPa.s/23°C
NCO-Gehalt: 18,2 %
freies HDI: 0,01 %
Hazen-Farbzahl: 30

## Patentansprüche

1. Verfahren zur Herstellung von Isocyanuratpolyisocyanaten durch Trimerisierung eines Teils der Isocyanatgruppen von (cyclo)aliphatischen Diisocyanaten in Gegenwart von die Trimerisierung von Isocyanatgruppen beschleunigenden Katalysatoren, Abbruch der Trimerisierungsreaktion beim jeweils gewünschten Trimerisierungsgrad und Entfernung von nicht umgesetztem Ausgangsdiisocyanat und von gegebenenfalls weiteren flüchtigen Bestandteilen, dadurch gekennzeichnet, daß man dem Reaktionsgemisch zu einem beliebigen Zeitpunkt vor Entfernung des überschüssigen Ausgangsdiisocyanats mindestens einen einwertigen, Estergruppen aufweisenden Alkohol des Molekulargewichts 146 bis 412 der allgemeinen Formel 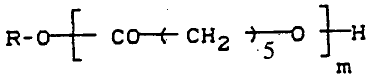 in welcher
R für einen, gegebenenfalls Ethersauerstoffatome aufweisenden, gegebenenfalls olefinisch ungesättigten aliphatischen Kohlenwasserstoffrest mit 1 - 18 Kohlenstoffatomen steht und
m für eine ganze oder im statistischen Mitteln gebrochene Zahl von 1 bis 2 steht,
in einer Menge von 1 bis 30 Gew.-%, bezogen auf das Gesamtgewicht des als Ausgangsmaterial eingesetzten Diisocyanats hinzufügt und mit einem Teil der vorliegenden Isocyanatgruppen unter Urethanbildung abreagieren läßt, wobei die Art und Mengenverhältnis der Reaktionspartner im übrigen so gewählt werden, daß in dem Reaktionsgemisch ohne Einbeziehung von gegebenenfalls mitverwendetem inerten Lösemittel nach beendeter Umsetzung noch mindestens 10 Gew.-% an freiem Ausgangsdiisocyanat vorliegen.

2. Verfahren gemäß Anspruch 1, dadurch gekennzeichnet, daß man als Ausgangsdiisocyanat 1,6-Diisocyanatohexan verwendet.

3. Verfahren gemäß Anspruch 1 und 2, dadurch gekennzeichnet, daß man als Esteralkohol einen solchen der in Anspruch 1 genannten allgemeinen Formel verwendet, für welchen
R für einen gesättigten aliphatischen Kohlenwasserstoffrest mit 1 bis 12 Kohlenstoffatomen steht,
m eine ganze oder gebrochene Zahl von 1 bis 2 steht.

4. Gemäß Anspruch 1 bis 3 erhaltene Isocyanuratpolyisocyanate.

5. Verwendung der gemäß Anspruch 1 bis 3 erhaltenen Isocyanuratpolyisocyanate, gegebenenfalls in mit Blockierungsmitteln für Isocyanatgruppen blockierter Form, zur Herstellung von Polyurethankunststoffen, insbesondere in Zweikomponenten-Polyurethanlacken.

## Claims

1. A process for preparing isocyanurate polyisocyanates by trimerising some of the isocyanate groups from (cyclo)aliphatic diisocyanates in the presence of the catalyst accelerating the trimerisation of isocyanate groups, interrupting the trimerisation reaction at the particular degree of trimerisation required and removing unreacted starting diisocyanate and optionally other volatile constituents, characterised in that, at any time before removing the excess starting diisocyanate, at least one monohydric alcohol containing ester groups, with a molecular weight of 146 to 412 and of the general formula
R-O-[CO-(CH₂)₅-0]ₘ-H,
in which
R represents an optionally olefinically unsaturated aliphatic hydrocarbon group with 1 - 18 carbon atoms, optionally containing ether oxygen atoms, and
m represents an integer or, as a statistical average, a fractional number from 1 to 2
is added to the reaction mixture in an amount of 1 to 30 wt.%, with respect to the total weight of the diisocyanate used as starting material and is allowed to react with some of the isocyanate groups which are present to form a urethane, wherein the type and ratio by weight of the reaction partners is generally selected so that after completion of the reaction at least 10 wt% of free starting diisocyanate is still present, not including any inert solvent which may optionally be used.

2. A process according to Claim 1, characterised in that 1,6-diisocyanatohexane is used as the starting diisocyanate.

3. A process according to Claim 1 and 2, characterised in that the esteralcohol used is one with a general formula like the one mentioned in Claim 1, in which
R represents a saturated aliphatic hydrocarbon with 1 to 12 carbon atoms,
m represents an integer or a fractional number from 1 to 2.

4. Isocyanurate polyisocyanates obtained in accordance with Claims 1 to 3.

5. Use of the isocyanurate polyisocyanates obtained in accordance with Claims 1 to 3, optionally in blocked form using blocking agents for isocyanate groups, to prepare polyurethane plastic materials, in particular in two-component polyurethane lacquers.

## Revendications

1. Procédé pour la préparation de polyisocyanates d'isocyanurates par trimérisation d'une partie des groupes isocyanates de diisocyanates (cyclo)aliphatiques en présence de catalyseurs accélérant la trimérisation de groupes isocyanates, interruption de la réaction de trimérisation au degré de trimérisation à chaque fois souhaité et élimination du diisocyanate de départ n'ayant pas réagi et éventuellement d'autres constituants volatils, caractérisé en ce qu'on ajoute au mélange réactionnel à un instant quelconque avant l'élimination du diisocyanate de départ en excès au moins un alcool monovalent présentant des groupes esters du poids moléculaire de 146 à 412 de la formule générale 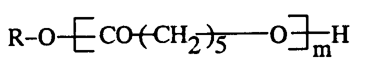 dans laquelle
R représente un résidu hydrocarboné aliphatique présentant éventuellement des atomes d'oxygène d'éther, éventuellement oléfiniquement insaturé, avec 1-18 atomes de carbone, et
m représente un nombre entier ou décimal en moyenne statistique de 1 à 2,
dans une quantité de 1 à 30 % en poids, rapportés au poids total du diisocyanate utilisé en tant que matériau de départ et on laisse réagir avec une partie des groupes isocyanates présents avec formation d'uréthane, le type et le rapport des quantités des partenaires réactionnels étant en outre choisi de telle sorte qu'encore au moins 10 % en poids de diisocyanate de départ libre sont présents dans le mélange réactionnel après la réaction terminée sans considérer le solvant inerte éventuellement co-utilisé.

2. Procédé selon la revendication 1, caractérisé en ce qu'on utilise en tant que diisocyanate de départ du 1,6-diisocyanatohexane.

3. Procédé selon les revendications 1 et 2, caractérisé en ce qu'on utilise en tant qu'alcool-ester un alcool-ester de la formule générale citée dans la revendication 1, pour lequel
R représente un résidu hydrocarboné aliphatique saturé avec de 1 à 12 atomes de carbone,
m représente un nombre entier ou décimal de 1 à 2.

4. Polyisocyanates d'isocyanurates obtenus selon l'une quelconque des revendications 1 à 3.

5. Utilisation des polyisocyanates d'isocyanurates obtenus selon l'une quelconque des revendications 1 à 3, éventuellement dans une forme bloquée avec des agents de blocage pour les groupes isocyanates, pour la préparation de matières synthétiques de polyuréthane, en particulier dans des laques de polyuréthane à deux constituants.
